(19) Europäisches Patentamt
European Patent Office
Office européen des brevets

(11) Veröffentlichungsnummer : **0 390 826 B1**

(12) **EUROPÄISCHE PATENTSCHRIFT**

(45) Veröffentlichungstag der Patentschrift : **19.11.92 Patentblatt 92/47**

(51) Int. Cl.$^5$ : **C07C 315/04,** C07C 305/00, C07C 303/24, C07B 45/02

(21) Anmeldenummer : **89900216.6**

(22) Anmeldetag : **29.11.88**

(86) Internationale Anmeldenummer : **PCT/EP88/01081**

(87) Internationale Veröffentlichungsnummer : **WO 89/05290 15.06.89 Gazette 89/13**

# (54) VERFAHREN ZUR HERSTELLUNG VON AMINOARYL-BETA-SULFATOETHYLSULFON-VERBINDUNGEN.

(30) Priorität : **11.12.87 DE 3742044 01.07.88 DE 3822231**

(43) Veröffentlichungstag der Anmeldung : **10.10.90 Patentblatt 90/41**

(45) Bekanntmachung des Hinweises auf die Patenterteilung : **19.11.92 Patentblatt 92/47**

(84) Benannte Vertragsstaaten : **AT BE CH DE FR GB IT LI NL SE**

(56) Entgegenhaltungen :
**DE-A- 3 026 808**
**DE-A- 3 340 114**

(56) Entgegenhaltungen :
**US-A- 4 334 076**
**CHEMIE INGENIEUR TECHNIK, Band 59, Nr. 2, 1987; R. HERBENER: "Staubarme Pulver durch Sprühtrocknen - Erfahrungen mit neuerenTechniken", siehe Seite 112-117**
**CHEMIE INGENIEUR TECHNIK, Band 51, Nr. 4, 1979; H. SCHUBERT: "Grundlagen des Agglomerierens", siehe Seite 266-277**

(73) Patentinhaber : **HOECHST AKTIENGESELLSCHAFT**
**Postfach 80 03 20**
**W-6230 Frankfurt am Main 80 (DE)**

(72) Erfinder : **RUPP, Walter**
**Robert-Schuman-Ring 106**
**W-6239 Kriftel (DE)**

**EP 0 390 826 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung liegt auf dem technischen Gebiet der Synthese von Zwischenprodukten, insbesondere für faserreaktive Farbstoffe.

Aminoaryl-β-sulfatoethylsulfon-Verbindungen werden heutzutage technisch im wesentlichen über zwei Verfahrensvarianten durch Sulfatisierung der entsprechenden Aminoaryl-β-hydroxyethylsulfon-Verbindung oder deren Acylamino-Derivate, wie deren Acetylamino-Verbindung, mittels einem Sulfatierungsmittel, wie beispielsweise konzentrierter wäßriger Schwefelsäure oder 100%iger Schwefelsäure oder Schwefeltrioxid enthaltender Schwefelsäure unter gleichzeitiger Hydrolyse einer gegebenenfalls vorhandenen Acylaminogruppe, hergestellt (s. auch Angewandte Chemie 74, 966 (1962)).

Bei der einen Verfahrensvariante wird die Amino- bzw. Acylaminoaryl-β-hydroxyethylsulfon-Verbindung in überschüssiger, etwa 95 bis 96 gew.-%ige oder 100 gew.-%ige Schwefelsäure oder Oleum mit unterschiedlichem Gehalt an freiem Schwefeltrioxid bei Raumtemperatur oder leicht erhöhter Temperatur, wie beispielsweise bei einer Temperatur zwischen 35 und 50°C, eingerührt. In der Regel wird ein Überschuß an Schwefelsäure bis zu 1000 Mol-% verwendet, der gleichzeitig als Lösemittel und zur Bindung des Reaktionswassers dient. Dieses Verfahren hat verschiedene Nachteile. Wird das Veresterungsprodukt durch Verdünnen mit Wasser und Eis entweder als inneres Salz ausgefällt oder nach einer Neutralisation als Neutralsalz isoliert, so entsteht in jedem Falle ein Prozeßabwasser mit hoher Salzbelastung. Wird das Veresterungsprodukt in Lösung weiterverarbeitet, wie beispielsweise für die Azofarbstoffherstellung in einer Diazotierungsreaktion, so müssen die Veresterungsreaktion und die Farbstoffherstellung im Produktionsablauf zeitlich koordiniert werden. Es entstehen durch Neutralisation der Farbstofflösungen, wie beispielsweise mittels Natriumcarbonat, Natronlauge oder Calciumcarbonat, erhebliche Salzmengen, die als hydratisiertes Natriumsulfat (Glaubersalz) aus den Farbstofflösungen bei tiefen Temperaturen auskristallisiert oder als Calciumsulfat ausgefällt werden und entweder weiterverwertet oder auf einer Abfalldeponie gelagert werden müssen.

Bei der anderen Verfahrensvariante wird die Veresterung mit stöchiometrischen Mengen an Schwefelsäure oder einem Überschuß von 2 bis 30 Mol-% an Schwefelsäure in einem sogenannten Kontakttrockner, wie beispielsweise in einer Trockenpfanne, bei einer Temperatur zwischen etwa 120°C und etwa 150°C, durchgeführt. Andere Apparate, mit deren Hilfe die Durchführung dieser Umsetzungen vorgeschlagen wurde, sind beheizte Kneter und Trockenbänder. Die Aminoaryl-β-hydroxyethylsulfon-Ausgangsverbindung bzw. Deren Acetylamino-Derivat wird in der Regel getrocknet und in Monohydrat oder konzentrierte Schwefelsäure eingerührt. Danach wird das Ganze auf die für das jeweilige Produkt optimale Reaktionstemperatur erhitzt und das Reaktionswasser und die freigesetzte Essigsäure bei Normaldruck oder vermindertem Druck ausgetrieben. Das Veresterungsprodukt fällt hierbei in feste: Form an. Auch diese Verfahrensweise besitzt Nachteile. So sind hierfür lange Reaktionszeiten von mehreren Stunden aufgrund der schlechten Wärmeübertragung von den beheizten Flächen des Reaktors in die Produktmasse, insbesondere wenn diese die feste Phase erreicht hat, erforderlich. Die Produktqualität leidet durch Nebenproduktbildung aufgrund stationärer thermischer Überbelastung im Zusammenhang mit den langen Reaktionszeiten. Das Reaktionsgemisch durchläuft vor dem Erreichen des festen Zustandes gegebenenfalls -in Abhängigkeit von jeweiligen Ausgangs-Aminoaryl-β-hydroxyethylsulfon - eine hochviskose, zähe Phase mit hoher mechanischer Beanspruchung der Apparate, die wiederum zu einer erhöhten Reparaturanfälligkeit führt. Außerdem werden die hierbei verwendeten Reaktoren, die üblicherweise aus austenitischen Stählen gefertigt sind, durch verdünnte Schwefelsäure, wie 20 bis 80 gew.-%ige Schwefelsäure, bei Temperaturen von oberhalb 120°C stark korrodiert, wobei ein Wandabtrag von bis zu 10 mm pro Jahr erfolgen kann. Die Endprodukte fallen in der Kornverteilung inhomogen an, und zwar gleichzeitig als Staub und als brockige Agglomerate, die eine zusätzliche Zerkleinerung in Brechern oder Mühlen notwendig machen. Darüberhinaus ist für deren Weiterverarbeitung von Nachteil, daß die als innere Salze erhaltenen Veresterungsprodukte in mechanisch harter Form anfallen und bei ihrer Auflösung in Wasser durch neutralisieren mit alkalisch wirkenden Mitteln langer Reaktionszeiten bedürfen.

Des weiteren sind aus der Deutschen Offenlegungsschrift Nr. 3 026 808 Verfahren bekannt, bei denen die Veresterung mit geringen Schwefelsäureüberschüssen durchgeführt wird, wobei die Entfernung der flüchtigen Reaktionsprodukte auf azeotropem Wege unter Verwendung organischer Lösemittel oder durch Verdampfen aus den gekneteten Gemisch bei Normaldruck oder unter reduziertem Druck oder in Trocknern mit Heißluftumwälzung durchgeführt wird. Die Reaktionszeiten betragen hierbei mindestens 4 Stunden (bspw. Beispiele 39 und 48), und weiterhin werden in der Regel Hilfsstoffe, wie Diatomeenerde, zugesetzt, die aus dem Endprodukt wieder entfernt werden müssen. Wesentlich bei diesem bekannten Verfahren der Deutschen Offenlegungsschrift Nr. 3 026 808 ist, daß man die Veresterung in der wäßrigen Schwefelsäure entweder zunächst bei Temperaturen um etwa 90 bis 125°C durchführt und anschließend das Wasser in einem Verdampfer entfernt (bspw. Beispiele 37, 40, 48 und 54) oder daß man während der Veresterungszeit von mehreren Stunden das Wasser langsam in einem Umwälztrockner entfernt (bspw. Beispiel 39). Neben diesen Nachteilen einer

langen Reaktionszeit mit zudem in der Regel hohen Schwefelsäureüberschüssen erfordern diese Verfahrensweisen wegen der starken Aggressivität der wäßrigen Schwefelsäure bei den angewendeten Temperaturen Apparaturen, die aus äußerst hochwertigen Werkstoffen hergestellt sind; handelsübliche Edelstähle werden unter diesen Bedingungen korrodiert.

Mit der vorliegenden Erfindung wurde nun gefunden, daß die vorgenannten Nachteile der bekannten Verfahrensweisen überraschenderweise vermieden und die Veresterungsreaktion und gleichzeitige Trocknung unter Verwendung geringer Mengen an Schwefelsäure durchgeführt werden können, wenn man die Sulfatisierungsreaktion von Aminoaryl-β-hydroxyethylsulfon-Verbindungen oder deren Acylamino-Derivate in der Weise durchführt, daß man eine Lösung oder Suspension oder Paste dieser Aminoaryl-β-hydroxyethylsulfon-Verbindung bzw. Deren Acylamino-Verbindung in 100 %iger Schwefelsäure oder in wäßriger Schwefelsäure mit einem Wassergehalt von bis zu 80 Gew.-% im Molverhältnis der β-Hydroxyethylsulfonyl-Ausgangsverbindung zu $H_2SO_4$ von 1:1 bis 1:1,05 nach deren Herstellung in den heißen Gasstrom (Luft, inertisierte Luft, Stickstoff) eines Wirbelschichtsprühgranulators oder Sprühtrockners oder Sprühtrockners mit integriertem Fließbett einsprüht und darin die Trocknung, die Veresterungsreaktion und die Hydrolyse einer gegebenenfalls vorhandenen Acylaminogruppe synchron bei einer Temperatur zwischen 100 und 200°C, vorzugsweise zwischen 110 und 180°C, durchführt.

Wie aus den obigen Angaben ersichtlich, kann die Schwefelsäure in stöchiometrischer Menge verwendet werden; bevorzugt setzt man sie in dem angegebenen Überschuß ein. Selbst bei diesen geringen Überschüssen an Schwefelsäure ist eine vollständige Veresterung durch die erfindungsgemäße Verfahrensweise gewährleistet und eine durch Versprühen verarbeitbare Lösung oder Suspension oder Paste aus der Ausgangsverbindung unter Schwefelsäure, die bevorzugt bei 80 bis 115°C hergestellt werden, herstellbar.

Die oben erwähnten, erfindungsgemäß eingesetzten Apparaturen werden im nachfolgenden als Konvektionstrockner bezeichnet. Solche Konvektionstrockner sind in der Literatur zahlreich beschrieben, wie beispielweise in Verfahrenstechnik 10, 758-763 (1976), in Chem. Ing. Techn. 51, 266-277 (1979) und Chem. Ing. Techn. 59, 112-117 (1987). Die erfindungsgemäß verwendeten Konvektionstrockner sind die in der Technik üblicherweise verwendeten Apparaturen, wie beispielsweise Wirbelschichtsprühgranulatoren, bei denen die Wirbelschicht pneumatisch und/oder mechanisch erzeugt wird, Fließbettrockner, Sprühtrockner sowie Sprühtrockner mit externem oder integriertem Fließbett und Spin-Flash-Trockner.

Arbeitsschemata, nach denen das erfindungsgemäße Verfahren mit solchen Konvektionstrocknern durchgeführt werden können, ergeben sich aus den beigefügten Figuren 1, 2 und 3:

Figur 1 = Arbeitsschema eines Wirbelschichtsprühgranulators;
Figur 2 = Arbeitsschema eines Sprühtrockners;
Figur 3 = Arbeitsschema eines Sprühtrockners mit integriertem Fließbett.

In diesen Figuren bedeuten:

(1) = Ventilator
(2) = Gaserhitzer
(3) = Siebboden
(4) = Wirbelschichtsprühgranulator
(5) = Zellenrad
(6) = Behälter mit Ausgangs-Reaktionsgemisch
(7) = Pumpe
(8) = Zerstäuberdüse (Einstoff- oder Zweistoffdüse) oder Zerstäuberscheibe
(9) = Gaserhitzer
(10) = Staubfilter
(11) = Kondensator
(12) = Behälter mit "Feingut" (Sulfato-Endprodukt)
(13) = Druckhaltungsvorrichtung mit Druckregler (13a)
(14) = Zellenrad
(15) = Sprühtrockner
(16) = Zyklon
(17) = Gaswäsche
(18) = Sammelbehälter für Granulat (Endprodukt)
(19) = Gaserhitzer
(20) = Sprühtrockner mit integriertem Fließbett
(21) = Feingutzuleitung
(22) = Ableitung des Gases durch die Turmdecke
(23) = Schieber
(24) = Feinguteinführung

(25) = Zerhacker
(26) = Rührwerk
(F) = Fließbett
(G) = Gas (Wärmeträgergas; Trocknungs- und/oder Wirbelgas)
(N) = Transportgas
(S) = Sprühgas
(K) = Kondensat.

Die verwendeten Gase sind in der Regel Luft oder Stickstoff oder ein Luft/Stickstoff-Gemisch mit bevorzugt geringem Sauerstoffgehalt.

Die Trocknung und die Veresterungsreaktion sowie die Hydrolyse einer gegebenenfalls vorhandenen Acylaminogruppe erfolgt zwischen 100 und 200°C, bevorzugt zwischen 110 und 180°C. Die Temperaturen werden in der Regel durch die Einsprühgeschwindigkeit des Reaktionsgemisches in den heißen Gasstrom eingestellt und konstant gehalten. Die Eingangstemperatur des Gases (G) liegt in der Regel zwischen 150 und 360°C, bevorzugt zwischen 180 und 300°C. Die Wahl der Gaseingangstemperatur kann bspw. von der Wahl des verwendeten Konvektionstrockners und/oder der Größe des gewählten Volumenstromes des Gases und/oder der Einsprühgeschwindigkeit und der Konzentration des Reaktionsgemisches abhängig sein. Die in den Beispielen angeführten maximalen Gaseingangstemperaturen sind experimentell angewandte Temperaturen, die keine Begrenzung für die technische Durchführung an den einzelnen Apparaturen bedeuten.

Als Schwefelsäure kann sowohl 100%ige als auch wäßrige Schwefelsäure mit einem Wassergehalt von bis zu 80 Gew.-% eingesetzt werden, je nach dem, ob die Ausgangsverbindung als technisch trockene oder als technisch wasserfeuchte Ware eingesetzt wird. In der Regel verwendet man wäßrige Schwefelsäure; bevorzugt setzt man, um die separate Trocknung der Ausgangsverbindung zu umgehen, die Ausgangsverbindung technisch wasserfeucht mit einem Feststoffgehalt von 50 bis 95 Gew.-% ein, wie sie aus dem Herstellprozeß anfällt, und verwendet Schwefelsäure mit einer Konzentration von 35 bis 95 Gew.-% $H_2SO_4$ und führt die Trocknung und die Veresterungsreaktion in einem Schritt durch.

In der Regel verfährt man erfindungsgemäß in der Weise, daß man von einer Lösung, Suspension oder Paste der Amino- bzw. Acylamino-aryl-β-hydroxyethylsulfon-Ausgangsverbindung in Schwefelsäure ausgeht, diese über eine Pumpe in den Reaktor bringt und dort mittels Düse oder Zerstäuberscheibe in den heißen Gasstrom (G) zerstäubt. Hierbei erfolgt aufgrund der gebildeten großen Produktoberfläche eine umgehende hydrolytische Abspaltung der Acylgruppe und gleichzeitige vollständige Trocknung und Veresterung (Sulfatisierung) zum Reaktionsprodukt. Vorteilhaft wird die Einspeisung des Reaktionsgemisches in den Gasstrom so ausgeführt, daß sich, sofern dies die Auslegung der Reaktionsapparatur zuläßt, in dem strömenden Gas ein Fließbett des Sulfato-Endproduktes bildet. Durch Ausbildung eines Fließbettes ergibt sich der Vorteil, daß das zerstäubte Reaktionsgemisch das Feingut des Fließbettes in dünner Schicht überzieht oder daß die feinen Teilchen agglomerieren und so ein staubfreies Granulat erzeugt wird. Die Bildung eines Granulats mit Korngrößen zwischen beispielsweise 100 und 3000 μm wird gegebenenfalls mittels Feingutdosierung und/oder Zerhacker gesteuert. Das Granulat kann aus der Reaktionsapparatur abgezogen und durch entsprechende Trennvorrichtungen in gegebenenfalls gewünschte Kornfraktionen zerlegt werden. Sehr feinkörnige Teilchen ("Feinkorn") und/oder zerkleinerte gröbere Agglomerate ("Überkorn") können wieder der Reaktionsapparatur bzw. dem Fließbett der Reaktionsapparatur zugeführt werden, um das Endprodukt in der gewünschten Granulatgröße zu erhalten. Das verdampfende Wasser aus den Einsatzprodukten und aus der Reaktion wird zusammen mit der abgespaltenen Säure einer gegebenenfalls vorhandenen Acylgruppe, bspw. der Essigsäure aus der Acetylgruppe, durch den heißen Gasstrom ausgetragen und in Kondensatoren durch Kühlung niedergeschlagen. Das vom Kondensat befreite Gas wird wieder auf die geforderte Eingangstemperatur aufgeheizt und erneut dem Reaktor als Wärmeträgergas zugeführt. Die Fließbettemperatur bzw. die Gasaustrittstemperatur bei Sprühtrocknern wird beispielsweise durch die Dosiergeschwindigkeit des Reaktionsgemisches eingestellt und konstant gehalten.

Es war überraschend und nicht vorhersehbar, daß im Temperaturbereich zwischen 100 und 200°C sehr kurze stationäre Verweilzeiten für die Reaktion ausreichen, um einen synchronen Ablauf der Wasserverdampfung aus dem versprühten Reaktionsgemisch und eine vollständige Veresterungsreaktion zu erzielen. Damit ist es möglich, in gleichem Maße, wie Ausgangs-Reaktionsgemisch eingesprüht wird, Reaktionsprodukt aus dem Reaktor abzuziehen. Die erfindungsgemäße Umsetzung kann folglich kontinuierlich durchgeführt werden. Es wird ein Endprodukt mit hohem Veresterungsgrad und hoher Produktqualität durch Unterdrückung der Bildung von Nebenprodukten erreicht, das zudem direkt als konfektioniertes Produkt, d.h. Feingranulat ohne oder ohne wesentliche Staubbelastung erhalten wird und wesentlich verbesserte Eigenschaften für dessen Weiterverarbeitung, wie beispielsweise eine leichtere Benetzbarkeit durch Wasser und eine höhere Lösegeschwindigkeit in Wasser bei der Neutralisation mittels Alkalien der als innere Salze vorliegenden Sulfatoverbindungen, aufweist.

Die vorliegende Erfindung betrifft insbesondere die Herstellung einer Amino-aryl-β-sulfatoethylsulfon-Verbindung entsprechend der allgemeinen Formel (1)

$$(HO_3SO - CH_2 - CH_2 - SO_2)_m - A - NH_2 \quad (1)$$

in welcher m die Zahl 1 oder 2 bedeutet und A ein Phenylen-oder Naphthylenrest ist, die durch 1, 2 oder 3, bevorzugt 1 oder 2, Substituenten aus der Gruppe Alkyl von 1 bis 4 C-Atomen, wie Methyl und Ethyl, Alkoxy von 1 bis 4 C-Atomen, wie Methoxy und Ethoxy, Halogen, wie Fluor und insbesondere Brom und Chlor, Carboxy und Hydroxy substituiert sein können,
unter Verwendung der entsprechenden Ausgangsverbindung der allgemeinen Formel (2)

$$(HO-CH_2-CH_2-SO_2)_m - A - \underset{\substack{| \\ R}}{N} - H \qquad (2)$$

in welcher m und A die obengenannten Bedeutungen haben und R für ein Wasserstoffatom oder eine Acylgruppe, insbesondere einer niederen Alkancarbonsäure, wie die Acetylgruppe, steht. Bevorzugt dient das Verfahren zur Sulfatisierung von Verbindungen der allgemeinen Formel (2), in welcher m die Zahl 1 ist und A einen 1,3- oder 1,4-Phenylenrest bedeutet, der unsubstituiert oder durch 1 Bromatom oder durch 1 oder 2 Methoxygruppen oder durch 1 Methylgruppe und 1 Methoxygruppe substituiert ist, oder A einen Naphthylenrest, bevorzugt 2,6- oder 2,8-Naphthylenrest, bedeutet.

Ausgangsverbindungen entsprechend der allgemeinen Formel (2), die erfindungsgemäß in deren Schwefelsäurehalbester (Sulfatoverbindungen) übergeführt werden können, sind beispielsweise 4-(β-Hydroxyethylsulfonyl)-anilin, 3-(β-Hydroxyethylsulfonyl)-anilin, 2-Methoxy-5-(β-hydroxyethylsulfonyl)-anilin, 4-Methoxy-5-(β-hydroxyethylsulfonyl)-anilin, 2-Hydroxy-5-(β-hydroxyethylsulfonyl)-anilin, 2-Methoxy-5-methyl-4-(β-hydroxyethylsulfonyl)-anilin, 2,5-Dimethoxy-4-(β-hydroxyethylsulfonyl)-anilin, 2-Brom-4-(β-hydroxyethylsulfonyl)-anilin, 6-(β-Hydroxyethylsulfonyl)-2-amino-naphthalin und 8-(β-Hydroxyethylsulfonyl)-2-amino-naphthalin sowie deren N-Acetyl-Derivate.

Die nachstehenden Beispiele dienen zur Erläuterung der Erfindung. Die angegebenen Teile sind Gewichtsteile, die Prozentangaben stellen Gewichtsprozente dar, sofern nicht anders vermerkt. Volumenteile stehen zu Gewichtsteilen im Verhältnis von Liter zu Kilogramm.

**Beispiele:**

Die Durchführung der erfindungsgemäßen Verfahrensweise wird in den nachstehenden Beispielen anhand der in den beigefügten Figuren 1, 2 und 3 beschriebenen Arbeitsschemata erläutert. Diese Arbeitsschemata stellen lediglich erläuternde Beispiele dar und sind als solche nicht erfindungswesentliche Bestandteile des erfindungsgemäßen Verfahrens.

**Funktionsprinzip des Wirbelschichtsprühgranulators (Figur 1):**

Der Ventilator (1) fördert das Gas (G) durch die Gaserhitzer (2), die beispielsweise elektrisch und/oder durch Heißdampf und/oder durch Gas oder Ölbrenner erhitzt werden können, und durch den Siebboden (3), über dem sich das für die Reaktion erwünschte Fließbett (F) bildet, das gegebenenfalls zusätzlich durch ein Rührwerk (26) bewegt werden kann, in den Granulator (4). Über das Zellenrad (5) und/oder durch Einblasen mit einem Transportgas (N) wird zunächst feingemahlenes Endprodukt, nämlich die Aminoaryl-β-sulfatoethylsulfon-Verbindung, im nachfolgenden als "Feingut" bezeichnet, und/oder ein inertes Feinmaterial, bspw. Aktivkohle, Kieselerden oder Salze, wie Natriumsulfat, aus dem Behälter (12) im Fließbett (F) vorgelegt, um zusammen mit dem heißen Wirbelgas (= Gas G) ein Fließbett entstehen zu lassen. Hat das Fließbett die Reaktionstemperatur von mindestens 100°C erreicht, wird die schwefelsaure Lösung, Suspension oder Paste der Aminoaryl-β-hydroxyethylsulfon-Ausgangsverbindung aus dem Behälter (6) mittels der Pumpe (7) über die Zweistoff-Düsen (8) in den Granulator (4) gefördert und mit dem Sprühgas S, das gegebenenfalls in einem Gaserhitzer (9) auf beispielsweise 90°C erwärmt wird, zerstäubt. Das zerstäubte Reaktionsgemisch überzieht das vorgelegte, feingemahlene Feingut im Fließbett in dünnen Schichten, so daß Trocknung und Reaktion aufgrund der großen, immer wieder neuen Oberfläche mit maximaler Geschwindigkeit ablaufen können. Das heiße Wirbelgas nimmt hierbei Wasser und gegebenenfalls flüchtige Nebenprodukte aus der Reaktion auf. Es wird im Staubfilter (10) von Produktstaub gereinigt, im Kondensator (11) von Wasser und flüchtigen Spaltprodukten befreit und über den Ventilator (1) und die Gaserhitzer (2) wieder dem Prozeß zugeführt. Das ständig in den Prozeß einfließende Sprühgas wird über eine Druckhaltungsvorrichtung (13) automatisch entlassen. Das erhaltene Endprodukt wird als Granulat aus dem Granulator (4) über das Zellenrad (14) ausgeschleust. -Zur gezielten Herstellung eines Granulats mit engem Kornspektrum kann, bspw. parallel mit dem Einsprühen des Reaktionsgemisches, Feingut aus dem Behälter (12) in das Fließbett eingeführt oder Feingut im Fließbett mit dem Zerhacker (25) erzeugt werden, indem der Zerhacker in Intervallen oder durchgehend läuft.

**Funktionsprinzip des Sprühtrockners (Figur 2):**

Über einen Gaserhitzer (2) wird mittels eines Ventilators (1) durch den Sprühturm (15) und den Zyklon (16) das heiße Gas (G) gesauft und anschließend in die Gaswäsche (17) gepreßt und dort von Feinstaub und flüchtigen Reaktionsprodukten gereinigt. Gleichzeitig wird die schwefelsaure Lösung, Suspension oder Paste der Aminoaryl-β-hydroxyethylsulfon-Ausgangsverbindung aus dem Behälter (6) beispielsweise mit der Pumpe (7) durch die Zweistoff-Düse (8) in den Sprühturm (15) gefördert und mittels des heißen Sprühgases (S), das gegebenenfalls erwärmt wird, versprüht (statt der Zweistoff-Düse (8) kann auch eine Einstoff-Düse oder ein Scheibenzerstäuber verwendet werden). Gleichzeitig mit dem Einsprühen des Reaktionsgemisches kann gegebenenfalls Feingut aus dem Behälter (12) über das Zellenrad (5) durch die Turmdecke eingeführt werden. Das versprühte Reaktionsgemisch wird im Gleichstrom durch das heiße Gas von Wasser und gegebenenfalls flüchtigen Nebenprodukten der Reaktion befreit, in den Zyklon (16) gefördert und dort im Behälter (18) als Granulat abgeschieden. Feinstaubteilchen des Endproduktes, die den Zyklon passieren, werden in der Gaswäsche (17) ausgewaschen. Im Pumpensumpf der Gaswäsche entsteht eine wäßrige Suspension der β-Sulfatoethylsulfon-Endverbindung, aus der sie isoliert werden kann. Unter Verwendung eines Staubfilters (10) und Kondensators (11) ist auch hier der Betrieb in Kreislauffahrweise, wie in Figur 1 beschrieben, möglich. Für diesen Fall kann die Gaswäsche entfallen.

**Funktionsprinzip des Sprühtrockners mit integriertem Fließbett (Figur 3)**

Mittels des Ventilators (1) wird das Gas (G) als Trocknungs-und Reaktionsgas über den Gaserhitzer (19a) in den Sprühturm (20) geblasen; desweiteren wird das Gas G über den Gaserhitzer (19b) erhitzt und über den Siebboden (3) als Wirbelgas in das sich ausbildende Fließbett (F) hineingepreßt. Ein Abzweig kann als Transportgas dazu eingesetzt werden, um Feingut beispielsweise aus dem Zyklon (16) oder einem Behälter (12) über eine Feinguteinführung (24) in den Reaktor (20), insbesondere dem Fließbett (F), zuzuführen, um zu Anfang der Umsectzung ein Fließbett auszubilden bzw. während des Einsprühens, gegebenenfalls unter zusätzlicher Anwendung des Zerhackers (25), die Granulatgröße zu steuern. Eine gegebenenfalls zusätzliche "Puderung" mit Feingut durch die Turmdecke kann, bspw. mit der in Figur 2 beschriebenen Feinguteinführung (21), erfolgen.

Aus dem Behälter (6) wird bspw. mittels der Pumpe (7) die schwefelsaure Lösung, Suspension oder Paste der Aminoaryl-β-hydroxyethylsulfon-Ausgangsverbindung in die Zweistoff-Düse (8) des Sprühtrockners (20) gefördert und dort mittels einem gegebenenfalls vorgewärmten Sprühgas (S) versprüht. Das entstehende Granulat des Endproduktes wird aus dem Sprühturm in den Behälter (18) ausgetragen. Das mit Wasser und gegebenenfalls flüchtigen Nebenprodukten der Reaktion beladene Gas verläßt den Sprühturm (20) über die Turmdecke (22) und wird im Zyklon (16) von noch nicht granuliertem Endprodukt und in der Gaswäsche (17) von Staub und flüchtigen Nebenprodukten gereinigt. Das im Zyklon (16) aus dem Gasstrom als Feingut abgetrennte Endprodukt kann über das Zellenrad (5) und die Zuführung (24) wieder der Granulierung im Fließbett zugeführt werden.

**Beispiele:**

**1. Herstellung von 4-(β-Sulfatoethylsulfonyl)-1-amino-benzol in Chargenfahrweise**

**1.a Herstellung der Reaktionsgemische**

Man rührt 4-(β-Hydroxyethylsulfonyl)-1-acetylamino-benzol technisch wasserfeucht oder technisch trocken in solch einer Menge wäßriger Schwefelsäure mit Prozentgehalten von beispielsweise 50 bis 95 % an, daß das Molverhältnis zwischen Sulfonylverbindung und Schwefelsäure beispielsweise 1:1 oder 1:1,02 oder 1:1,05 beträgt. Man erhält bei beispielsweise 100°C eine Lösung oder bei 20 bis 25°C eine Suspension, die in den Konvektionstrockner eingespeist werden. Bevorzugt wird mit Reaktionsgemischen gearbeitet, die 50 bis 66 % 4-(β-Hydroxyethylsulfonyl)-1-acetylamino-benzol und 21 bis 28 % Schwefelsäure (100 %ig gerechnet) enthalten.

### 1.b Verfahrensbeispiele

### 1.b.1 Verfahrensprinzip

In einem Wirbelschichtsprühgranulator (beispielsweise gemäß Figur 1) baut man mit dem Gas (G) und mit Feingut des Endproduktes, nämlich 4-(β-Sulfatoethylsulfonyl)-1-amino-benzol mit einer Korngröße von zum Beispiel kleiner oder gleich 100 μm ein Fließbett auf. Die Eingangstemperatur des Gases (G) wählt man beispielsweise wie in den Beispielen 1.b.2 bis 1.b.5 beschrieben und hält die Fließbettemperatur (= Reaktionstemperatur) wie in den Beispielen 1.b.2 bis 1.b.5 beschrieben konstant, indem man stetig eine der unter 1.a hergestellten Lösungen oder Suspensionen der Sulfonyl-Ausgangsverbindung in das Fließbett einsprüht. Im Fließbett entsteht hierbei ein Granulat, dessen Korngröße man durch Einschleusen von Feingut des Sulfato-Endproduktes und/oder durch Zerkleinern mittels Zerhacker (25) steuert und so ein Granulat von beispielsweise 100 bis 800 μm oder 100 bis 2000 μm oder 100 bis 3000 μm erzeugt. Der Prozeß wird nach beispielsweise 2 oder 3 oder 4 oder 5 Stunden unterbrochen, nämlich dann, wenn die Granulatmenge im Fließbett so groß geworden ist, daß das optimale "Fließen" der Granulatkörner nicht mehr aufrechterhalten werden kann. Dann wird das Fließbett entleert und der Prozeß beginnt von neuem.

**Beispiel 1.b.2**

Man verfährt, wie unter dem Verfahrensprinzip 1.b.1 beschrieben, unter Anwendung einer Eingangstemperatur des Gases (G) von 200°C und einer Fließbettemperatur (= Reaktionstemperatur) von 120°C. Man erhält ein Granulat mit der Zusammensetzung und den Qualitätsmerkmalen, wie sie unter 1.c beschrieben sind.

**Beispiel 1.b.3**

Man verfährt, wie unter dem Verfahrensprinzip 1.b.1 beschrieben, unter Anwendung einer Eingangstemperatur des Gases (G) von 200°C und einer Fließbettemperatur (= Reaktionstemperatur) von 150°C. Man erhält ein Granulat mit der Zusammensetzung und den Qualitätsmerkmalen, wie sie unter 1.c beschrieben sind.

**Beispiel 1.b.4**

Man verfährt, wie unter dem Verfahrensprinzip 1.b.1 beschrieben, unter Anwendung einer Eingangstemperatur des Gases (G) von 240°C und einer Fließbettemperatur (= Reaktionstemperatur) von 170°C. Man erhält ein Granulat mit der Zusammensetzung und den Qualitätsmerkmalen, wie sie unter 1.c beschrieben sind.

**Beispiel 1.b.5**

Man verfährt, wie unter dem Verfahrensprinzip 1.b.1 beschrieben, unter Anwendung einer Eingangstemperatur des Gases (G) von 170°C und einer Fließbettemperatur (= Reaktionstemperatur) von 115°C. Man erhält ein Granulat mit der Zusammensetzung und den Qualitätsmerkmalen, wie sie unter 1.c beschrieben sind.

### 1.c Ergebnisse

Anhand von Analysen von Proben, die während des Einsprühens der schwefelsauren Lösung der Sulfonyl-Ausgangsverbindung aus dem Fließbett entnommen wurden, wurde festgestellt, daß die Veresterungsreaktion (Sulfatierung) während des Einsprühens spontan und praktisch vollständig abläuft, so daß eine Nachreaktionszeit ("Temperzeit") nicht erforderlich ist. Dies wird zusätzlich bestätigt durch Analysen von Granulatproben, die im Fließbett bei der gewählten Reaktionstemperatur einer "Temperzeit" von bis zu 4 Stunden unterworfen waren und deren Produktzusammensetzung sich hinsichtlich des Gehaltes an Sulfato-Endprodukt nicht mehr veränderte. Die Granulate der Beispiele 1.b.2 bis 1.b.5 enthielten durchschnittlich

98 % 4-(β-Sulfatoethylsulfonyl)-1-amino-benzol,
0,1-0,5 % 4-(β-Hydroxyethylsulfonyl)-1-acetylamino-benzol und
0,2-0,5 % 4-(β-Hydroxyethylsulfonyl)-1-amino-benzol.

Die praktisch staubfreien Granulate zeichnen sich gegenüber Produkten, die in Kontakttrocknern, beispielsweise einer Trockenpfanne, hergestellt wurden, durch eine höhere Lösegeschwindigkeit in wäßriger Suspension bei Zugabe von calcinierter Soda bis zu einem pH-Wert von 7 aus.

**2. Herstellung von 4-(β-Sulfatoethylsulfonyl)-1-amino-benzol in kontinuierlicher Fahrweise**

**2.a Herstellung der Reaktionsgemische**

Die Reaktionsgemische werden wie unter 1.a beschrieben hergestellt.

**2.b Verfahrensbeispiele**

Wie im Verfahrensprinzip 1.b.1 beschrieben, wird mit dem Gas (G) und mit Feingut des Sulfato-Endproduktes ein Fließbett aufgebaut. Die angewendeten Eingangstemperaturen des Gases (G) und die Fließbettemperaturen werden wie in den Beispielen 1.b.2 bis 1.b.4 beschrieben gewählt.
Im Unterschied zur Verfahrensweise der chargenweisen Herstellung wird bei der kontinuierlichen Fahrweise das erzeugte Granulat parallel mit dem Einsprühen der Reaktionsgemische, wie sie unter 1.a beschrieben sind, kontinuierlich mit dem Zellenrad (14) ausgeschleust, so daß die im Fließbett vorhandene Granulatmenge konstant bleibt. Die Korngröße des Granulats wird wie bei der Chargenfahrweise durch Eindosieren von "Feingut" (Sulfato-Endprodukt) und/oder durch Zerkleinern des Granulats mit dem Zerhacker (25) gesteuert, so daß der Prozeß aufgrund der Granulatmenge im Fließbett und mit dem gleichbleibenden Kornspektrum im optimalen "Wirbelbereich" abläuft.
Die ausgeübte kontinuierliche Fahrweise ist bis zu 98 Stunden lang ohne Unterbrechung durchgeführt worden.

**2.c Ergebnisse**

Die Analysen und Untersuchungen der zunächst stündlich, dann in größeren zeitlichen Abständen entnommenen Granulatproben zeigen die Qualitätsmerkmale, wie sie unter 1.c beschrieben sind.

**3. Herstellung von 4-(β-Sulfatoethylsulfonyl)-1-amino-benzol in einem Sprühtrockner (beispielsweise gemäß Figur 2)**

**3.a Herstellung der Reaktionsgemische**

Die Reaktionsgemische werden angerührt, wie unter 1.a beschrieben.

**3.b Verfahrensbeispiele**

**3.b.1 Verfahrensprinzip**

Das Gas (G) wird, wie in den Beispielen 3.b.2 bis 3.b.5 beschrieben, auf die Eintrittstemperatur aufgeheizt. In den heißen Gasstrom versprüht man die Reaktionsgemische, die unter 1.a beschrieben sind, kontinuierlich ein, und zwar mit einer solchen Geschwindigkeit, daß die in den Beispielen 3.b.2 bis 3.b.5 beschriebenen Ausgangstemperaturen des Gases (G) konstant bleiben. Gleichzeitig mit dem Einsprühen des Reaktionsgemisches schleust man gegebenenfalls "Feingut" (Sulfato-Endprodukt, Korngröße bspw. kleiner oder gleich 100 μm) aus dem Behälter (12) mit dem Zellenrad (5) durch die Turmdecke in den Sprühtrockner ein.

**Beispiel 3.b.2**

Man verfährt, wie unter dem Verfahrensprinzip 3.b.1 beschrieben, unter Anwendung einer Eingangstemperatur des Gases (G) von 220°C und einer Gasaustrittstemperatur von 150°C. Man erhält ein Granulat mit der Zusammensetzung und den Qualitätsmerkmalen, wie sie unter 1.c beschrieben sind.

**Beispiel 3.b.3**

Man Verfährt, wie unter dem Verfahrensprinzip 3.b.1 beschrieben, unter Anwendung einer Eingangstemperatur des Gases (G) von 260°C und einer Gasaustrittstemperatur von 200°C. Man erhält ein Granulat mit der Zusammensetzung und den Qualitätsmerkmalen, wie sie unter 1.c beschrieben sind.

**Beispiel 3.b.4**

Man verfährt, wie unter dem Verfahrensprinzip 3.b.1 beschrieben, unter Anwendung einer Eingangstemperatur des Gases (G) von 230°C und einer Gasaustrittstemperatur von 180°C. Man erhält ein Granulat mit der Zusammensetzung und den Qualitätsmerkmalen, wie sie unter 1.c beschrieben sind.

**Beispiel 3.b.5**

Man verfährt, wie unter dem Verfahrensprinzip 3.b.1 beschrieben, unter Anwendung einer Eingangstemperatur des Gases (G) von 200°C und einer Gasaustrittstemperatur von 125°C. Man erhält ein Granulat mit der Zusammensetzung und den Qualitätsmerkmalen, wie sie unter 1.c beschrieben sind.

**4. Herstellung von 4-(β-Sulfatoethylsulfonyl)-1-amino-benzol in einem Sprühtrockner mit integriertem Fließbett (beispielsweise gemäß Figur 3)**

**4.a Herstellung der Reaktionsgemische**

Die Reaktionsgemische werden angerührt, wie unter 1.a beschrieben.

**4.b Verfahrensbeispiele**

**4.b.1 Verfahrensprinzip**

Das Verfahren läuft ab, wie es im Funktionsprinzip des Sprühtrockners mit integriertem Fließbett beschrieben ist. Das Trocknungs- und Wärmeträgergas wird im Gaserhitzer (19a) auf die in den Beispielen 4.b.2 bis 4.b.5 angegebenen Eintrittstemperaturen aufgeheizt. Das für das Fließbett benötigte Wirbelgas wird im Gaserhitzer (19b) auf die in den Beispielen 4.b.2 bis 4.b.5 angegebenen Fließbettemperaturen aufgeheizt. Die Temperatur (Ausgangstemperatur) des mit flüchtigen Reaktionsprodukten und Produktstaub beladenen Gasstromes (22), der den Trockner durch die Turmdecke verläßt, wird, wie in den Beispielen 4.b.2 bis 4.b.5 angegeben, eingestellt und konstant gehalten, indem man das Reaktionsgemisch mit entsprechender Geschwindigkeit kontinuierlich in den Sprühtrockner eindosiert (einsprüht). Die Granulatgröße steuert man durch Einschleusen von "Feingut" (Sulfato-Endprodukt, Korngröße kleiner oder gleich 100 μm) über die Feingutzuführung (24) und/oder die Feingutzuleitung (21) und/oder durch Zerkleinerung mit dem Zerhacker (25).

**Beispiel 4.b.2**

Man verfährt, wie unter dem Verfahrensprinzip 4.b.1 beschrieben, unter Anwendung einer Eingangstemperatur des Gases (G) von 300°C, einer Fließbettemperatur von 135°C und einer Gasaustrittstemperatur von 150°C. Man erhält ein Granulat mit der Zusammensetzung und den Qualitätsmerkmalen, wie sie unter 1.c beschrieben sind.

**Beispiel 4.b.3**

Man verfährt, wie unter dem Verfahrensprinzip 4.b.1 beschrieben, unter Anwendung einer Eingangstemperatur des Gases (G) von 350°C und einer Fließbettemperatur von 190°C und einer Gasaustrittstemperatur von 200°C. Man erhält ein Granulat mit der Zusammensetzung und den Qualitätsmerkmalen, wie sie unter 1.c beschrieben sind.

**Beispiel 4.b.4**

Man verfährt, wie unter dem Verfahrensprinzip 4.b.1 beschrieben, unter Anwendung einer Eingangstemperatur des Gases (G) von 260°C und einer Fließbettemperatur von 160°C und einer Gasaustrittstemperatur von 160°C.

Man erhält ein Granulat mit der Zusammensetzung und den Qualitätsmerkmalen, wie sie unter 1.c beschrieben sind.

**Beispiel 4.b.5**

Man verfährt, wie unter dem Verfahrensprinzip 4.b.1 beschrieben, unter Anwendung einer Eingangstemperatur des Gases (G) von 210°C und einer Fließbettemperatur von 140°C und einer Gasaustrittstemperatur von 160°C. Man erhält ein Granulat mit der Zusammensetzung und den Qualitätsmerkmalen, wie sie unter 1.c

beschrieben sind.

## 5. Herstellung von 2-Brom-4-(β-sulfatoethylensulfonyl)-anilin in Chargenfahrweise

### 5.a Herstellung der Reaktionsgemische

Man rührt 2-Brom-4-(β-hydroxyethylsulfonyl)-anilin technisch wasserfeucht oder technisch trocken in solch einer Menge wäßriger Schwefelsäure mit Prozentgehalten von beispielsweise 30 bis 95 % an, daß das Molverhältnis zwischen Sulfonylverbindung und Schwefelsäure beispielsweise 1:1 oder 1:1,02 oder 1;1,05 beträgt. Man erhält bei beispielsweise 80-90°C eine Lösung oder bei 20-25°C eine Suspension, die in den Konvektionstrockner eingespeist werden.

Bevorzugt wird mit Reaktionsgemischen gearbeitet, die 49-68 % 2-Brom-4-(β-hydroxyethylsulfonyl)-anilin und 17,5 - 25 % Schwefelsäure (als 100 %ig gerechnet) enthalten.

### 5.b Verfahrensbeispiele

#### 5.b.1 Verfahrensprinzip

In einem Wirbelschichtsprühgranulator (beispielsweise gemäß Figur 1) baut man mit dem Gas (G) und mit Feingut des Endproduktes, nämlich 2-Brom-4-(β-sulfatoethylsulfonyl)-anilin mit einer Korngröße von z.B. kleiner oder gleich 100 µm, ein Fließbett auf. Die Eingangstemperatur des Gases (G) wählt man beispielsweise wie in den Beispielen 5.b.2 bis 5.b.5 beschrieben und hält die Fließbettemperatur (= Reaktionstemperatur) wie in den Beispielen 5.b.2 bis 5.b.5 beschrieben konstant, indem man stetig eine der unter 5.a hergestellten Lösungen oder Suspensionen der Sulfonyl-Ausgangsverbindung in das Fließbett einsprüht. Im Fließbett entsteht hierbei ein Granulat, dessen Korngröße man durch Einschleusen von Feingut des Sulfato-Endproduktes und/oder durch Zerkleinern mittels Zerhacker (25) steuert und so ein Granulat von beispielsweise 100 bis 800 µm oder 100 bis 2000 µm oder 100 bis 3000 µm erzeugt. Der Prozeß wird nach beispielsweise 2 oder 3 oder 4 oder 5 Stunden unterbrochen, nämlich dann, wenn die Granulatmenge im Fließbett so groß geworden ist, daß das optimale "Fließen" der Granulatkörner nicht mehr aufrecherhalten werden kann. Dann wird das Fließbett entleert und der Prozeß beginnt von neuem.

#### Beispiel 5.b.2.

Man verfährt, wie unter dem Verfahrensprinzip 5.b.1 beschrieben, unter Anwendung einer Eingangstemperatur des Gases (G) von 200°C und einer Fließbettemperatur (= Reaktionstemperatur) von 140°C. Man erhält ein Granulat mit der Zusammensetzung und den Qualitätsmerkmalen, wie sie unter 5.c beschrieben sind.

#### Beispiel 5.b.3

Man verfährt, wie unter dem Verfahrensprinzip 5.b.1 beschrieben, unter Anwendung einer Eingangstemperatur des Gases (G) von 230°C und einer Fließbettemperatur (= Reaktionstemperatur) von 160°C. Man erhält ein Granulat mit der Zusammensetzung und den Qualitätsmerkmalen, wie sie unter 5.c beschrieben sind.

#### Beispiel 5.b.4

Man verfährt, wie unter dem Verfahrensprinzip 5.b.1 beschrieben, unter Anwendung einer Eingangstemperatur des Gases (G) von 170°C und einer Fließbettemperatur (= Reaktionstemperatur) von 115°C. Man erhält ein Granulat mit der Zusammensetzung und den Qualitätsmerkmalen, wie sie unter 5.c beschrieben sind.

#### Beispiel 5.b.5

Man verfährt, wie unter dem Verfahrensprinzip 5.b.1 beschrieben, unter Anwendung einer Eingangstemperatur des Gases (G) von 210°C und einer Fließbettemperatur (= Reaktionstemperatur) von 130°C. Man erhält ein Granulat mit der Zusammensetzung und den Qualitätsmerkmalen, wie sie unter 5.c beschrieben sind.

### 5.c Ergebnisse

Anhand der Analysen von Granulatproben, die während des Einsprühens der schwefelsauren Lösung oder

Suspension der Sulfonyl-Ausgangsverbindung aus dem Fließbett entnommen wurden, wurde festgestellt, daß die Veresterungsreaktion (Sulfatierung) während des Einsprühens spontan und praktisch vollständig abläuft, so daß eine Nachreaktionszeit ("Temperzeit") nicht erforderlich ist. Dies wurde zusätzlich bestätigt durch Analysen von Granulatproben, die im Fließbett bei der gewählten Reaktionstemperatur einer "Temperzeit" von bis zu 2 Stunden unterworfen waren und deren Produktzusammensetzung sich hinsichtlich des Gehaltes an Sulfato-Endprodukt nicht mehr veränderte. Die Granulate der Beispiele 5.b.2 bis 5.b.5 enthielten durchschnittlich

96-98 % 2-Brom-4-(β-sulfatoethylsulfonyl)-anilin
1,5-2,5 % 2-Brom-4-(β-hydroxyethylsulfonyl)-anilin und
1-1,5 % 4-(β-Sulfatoethylsulfonyl)-anilin.

Die praktisch staubfreien Granulate zeichnen sich gegenüber Produkten, die in Kontakttrocknern, beispielsweise einer Trockenpfanne, hergestellt wurden, durch eine höhere Lösegeschwindigkeit in wäßriger Suspension bei Zugabe von calcinierter Soda bis zu einem pH-Wert von 7 aus.

**6. Herstellung von 2-Brom-4-(β-sulfatoethylsulfonyl)-anilin in kontinuierlicher Fahrweise**

**6.a Herstellung der Reaktionsgemische**

Die Reaktionsgemische werden wie unter 5.a beschrieben hergestellt.

**6.b Verfahrensbeispiele**

Wie im Verfahrensprinzip 5.b.1 beschrieben, wird mit dem Gas (G) und mit Feingut des Sulfato-Endproduktes ein Fließbett aufgebaut.
Die angewendete Eingangstemperatur des Gases (G) und die Fließbettemperatur werden bspw. bei 200°C/140°C bzw. 210°C/130°C bzw. 190°C/150°C gewählt.
Im Unterschied zur Verfahrensweise der chargenweisen Herstellung wird bei der kontinuierlichen Fahrweise das erzeugte Granulat parallel mit dem Einsprühen der Reaktionsgemische, wie sie unter 5.a beschrieben sind, kontinuierlich mit dem Zellenrad (14) ausgeschleust, so daß die im Fließbett vorhandene Granulatmenge konstant bleibt. Die Korngröße des Granulates wird wie bei der Chargenfahrweise durch Eindosieren von "Feingut" (Sulfato-Endprodukt) und/ oder durch Zerkleinern des Granulates mit dem Zerhacker (25) gesteuert, so daß der Prozeß aufgrund der konstanten Granulatmenge im Fließbett und mit dem gleichbleibenden Kornspektrum im optimalen "Wirbelbereich" abläuft.

**6.c Ergebnisse**

Anhand der Analysen von Granulatproben, die während des Einsprühens der schwefelsauren Lösung oder Suspension der Sulfonyl-Ausgangsverbindung aus den Fließbett entnommen wurden, wurde festgestellt, daß die Veresterungsreaktion (Sulfatierung) während des Einsprühens spontan und praktisch vollständig abläuft, so daß eine Nachreaktionszeit ("Temperzeit") nicht erforderlich ist. Dies wurde zusätzlich bestätigt durch Analysen von Granulatproben, die im Fließbett bei der gewählten Reaktionstemperatur einer "Temperzeit" von bis zu 3 Stunden unterworfen waren und deren Produktzusammensetzung sich hinsichtlich des Gehaltes an Sulfato-Endprodukt nicht mehr veränderten. Die Granulate der Beispiele mit den unter 6.b angeführten Gaseintritts- bzw. Fließbettemperaturen enthielten durchschnittlich

98 % 2-Brom-4-(β-sulfatoethylsulfonyl)-anilin
1 % 2-Brom-4-(β-hydroxyethylsulfonyl)-anilin und
1 % 4-(β-Sulfatoethylsulfonyl)-anilin, das aus

4-(β-Hydroxyethylsulfonyl)-anilin, einer Verunreinigng der Ausgangsverbindung, entsteht.

Die praktisch staubfreien Granulate zeichnen sich gegenüber Produkten, die in Kontakttrocknern beispielsweise einer Trockenpfanne hergestellt wurden, durch eine höhere Lösegeschwindigkeit in wäßriger Suspension bei Zugabe von calcinierter Soda bis zu einem pH-Wert von 7 aus.

**7. Herstellung von 2,5-Dimethoxy-4-(β-sulfatoethylsulfonyl)-anilin**

**7.a Herstellung der Reaktionsgemische**

Man rührt 2,5-Dimethoxy-4-(β-hydroxyethylsulfonyl)-1-acetylamino -benzol technisch wasserfeucht oder technisch trocken in solch einer Menge wäßriger Schwefelsäure mit Prozentgehalten von 20 bis 95 % an, daß

das Molverhältnis zwischen Sulfonylverbindung und Schwefelsäure beispielsweise 1:1 oder 1:1,02 oder 1;1,04 beträgt. Man erhält bei beispielsweise 90 bis 100°C eine Lösung oder bei 20 bis 25°C eine Suspension, die in den Konvektionstrockner eingespeist werden. Bevorzugt wird mit Reaktionsgemischen gearbeitet, die 50 bis 68 % 2,5-Dimethoxy-4-(β-hydroxyethylsulfonyl)-1-acetylamino-benzol und 16 bis 23 % Schwefelsäure (als 100 %ig gerechnet) enthalten.

**7.b Verfahrensbeispiele**

**7.b.1 Verfahrensprinzip**

In einem Wirbelschichtsprühgranulator (beispielsweise gemäß Figur 1) baut man mit dem Gas (G) und mit Feingut des Endproduktes, nämlich 2,5-Dimethoxy-4-(β-sulfatoethylsulfonyl)-anilin mit einer Korngröße von z.B. kleiner oder gleich 100 μm, ein Fließbett auf. Die Eingangstemperatur des Gases (G) wählt man beispielsweise wie in den Beispielen 7.b.2 bis 7.b.5 beschrieben und hält die Fließbettemperatur (= Reaktionstemperatur), wie in den Beispielen 7.b.2 bis 7.b.5 beschrieben, konstant, indem man stetig eine der unter 7.a hergestellten Lösungen oder Suspensionen der Sulfonyl-Ausgangsverbindung in das Fließbett einsprüht. Im Fließbett entsteht hierbei ein Granulat, dessen Korngröße man durch Einschleusen von Feingut des Sulfato-Endproduktes und/oder durch Zerkleinern mittels Zerhacker (25) steuert und so ein Granulat von beispielsweise 100 bis 800 μm oder 100 bis 2000 μm oder 100 bis 3000 μm erzeugt.

**Beispiel 7.b.2**

Man verfährt, wie unter dem Verfahrensprinzip 7.b.1 beschrieben, unter Anwendung einer Eingangstemperatur des Gases (G) von 200°C und einer Fließbettemperatur (= Reaktionstemperatur) von 130 ± 5°C. Man erhält ein Granulat mit der Zusammensetzung und den Qualitätsmerkmalen, wie sie unter 7.c beschrieben sind.

**Beispiel 7.b.3**

Man verfährt, wie unter dem Verfahrensprinzip 7.b.1 beschrieben, unter Anwendung einer Eingangstemperatur des Gases (G) von 220°C und einer Fließbettemperatur (= Reaktionstemperatur) von. 165 ± 5°C. Man erhält ein Granulat mit der Zusammensetzung und den Qualitätsmerkmalen, wie sie unter 7.c beschrieben sind.

**Beispiel 7.b.4**

Man verfährt, wie unter dem Verfahrensprinzip 7.b.1 beschrieben, unter Anwendung einer Eingangstemperatur des Gases (G) von 180°C und einer Fließbettemperatur (= Reaktionstemperatur) von 145°C. Man erhält ein Granulat mit der Zusammensetzung und den Qualitätsmerkmalen, wie sie unter 7.c beschrieben sind.

**Beispiel 7.b.5**

Man verfährt, wie unter dem Verfahrensprinzip 7.b.1 beschrieben, unter Anwendung einer Eingangstemperatur des Gases (G) von 220°C und einer Fließbettemperatur (= Reaktionstemperatur) von 150°C Man erhält ein Granulat mit der Zusammensetzung und den Qualitätsmerkmalen, wie sie unter 7.c beschrieben sind.

**7.c Ergebnisse**

Anhand der Analysen von Granulatproben, die während des Einsprühens der schwefelsauren Lösung oder Suspension der Sulfonyl-Ausgangsverbindung aus dem Fließbett entnommen wurden, wurde festgestellt, daß die Veresterungsreaktion (Sulfatierung) während des Einsprühens spontan und praktisch vollständig abläuft, so daß eine Nachreaktionszeit ("Temperzeit") nicht erforderlich ist. Dies wurde zusätzlich bestätigt durch Analysen von Granulatproben, die im Fließbett bei der gewählten Reaktionstemperatur einer "Temperzeit" von bis zu 2 Stunden unterworfen waren und deren Produktzusammensetzung sich hinsichtlich des Gehaltes an Sulfato-Endprodukt nicht mehr veränderte. Die Granulate der Beispiele 7.b.2 bis 7.b.5 enthielten durchschnittlich

> 96 % 2,5-Dimethoxy-4-(β-sulfatoethylsulfonyl)-anilin,
< 2,5 % 2,5-Dimethoxy-4-(β-hydroxyethylsulfonyl)-1-acetylamino -benzol und
0,3 % 2,5-Dimethoxy-4-(β-hydroxyethylsulfonyl)anilin.

Die praktisch staubfreien Granulate zeichnen sich gegenüber Produkten, die in Kontakttrocknern, beispielsweise einer Trockenpfanne, hergestellt wurden, durch eine höhere Lösegeschwindigkeit in wäßriger Suspension bei Zugabe von calcinierter Soda bis zu einem pH-Wert von 7 aus.

## 8. Herstellung von 2-Methoxy-5-methyl-4-(β-sulfatoethylsulfonyl)-anilin

### 8.a Herstellung der Reaktionsgemische

Man rührt 2-Methoxy-5-methyl-4-(β-hydroxyethylsulfonyl)-1-acetylamino-benzol technisch wasserfeucht oder technisch trocken in solch einer Menge wäßriger Schwefelsäure mit Prozentgehalten von 10 bis 95 % an, daß das Molverhältnis von Sulfonylverbindung und Schwefelsäure beispielsweise 1:1 oder 1:1,02 oder 1:1,05 beträgt. Man erhält bei beispielsweise 90 bis 100°C eine Lösung oder bei 20 bis 25°C eine Suspension, die in den Konvektionstrockner eingespeist werden.
Bevorzugt wird mit Reaktionsgemischen gearbeitet, die 37 bis 55 % 2-Methoxy-5-methyl-4-(β-hydroxyethylsulfonyl)-1-acetylamino-benzol und 13 bis 20 % Schwefelsäure (als 100 %ig gerechnet) enthalten.

### 8.b Verfahrensbeispiele

#### 8.b.1 Verfahrensprinzip

In einem Wirbelschichtsprühgranulator (beispielsweise gemäß Figur 1) baut man mit dem Gas (G) und mit Feingut des Endproduktes, nämlich 2-Methoxy-5-methyl-4-(β-sulfatoethylsulfonyl)-anilin mit einer Korngröße von z.B. kleiner oder gleich 100 µm, ein Fließbett auf. Die Eingangstemperatur des Gases (G) wählt man beispielsweise wie in den Beispielen 8.b.2 bis 8.b.5 beschrieben und hält die Fließbettemperatur (= Reaktionstemperatur), wie in den Beispielen 8.b.2 bis 8.b.5 beschrieben, konstant, indem man stetig eine der unter 8.a hergestellten Lösungen oder Suspensionen der Sulfonyl-Ausgangsverbindung in das Fließbett einsprüht. Im Fließbett entsteht hierbei ein Granulat, dessen Korngröße man durch Einschleusen von Feingut des Sulfato-Endproduktes und/oder durch Zerkleinern mittels Zerhacker (25) steuert und so ein Granulat von beispielsweise 100 bis 800 µm oder 100 bis 200 µm oder 100 bis 3000 µm erzeugt.

#### Beispiel 8.b.2

Man verfährt, wie unter dem Verfahrensprinzip 8.b.1 beschrieben, unter Verwendung einer Eingangstemperatur des Gases (G) von 220°C und einer Fließbettemperatur (= Reaktionstemperatur) von 165°C. Man erhält ein Granulat mit der Zusammensetzung und den Qualitätsmerkmalen, wie sie unter 8.c beschrieben sind.

#### Beispiel 8.b.3

Man verfährt, wie unter dem Verfahrensprinzip 8.b.1 beschrieben, unter Anwendung einer Eingangstemperatur des Gases (G) von 200°C und einer Fließbettemperatur (= Reaktionstemperatur) von 145°C. Man erhält ein Granulat mit der Zusammensetzung und den Qualitätsmerkmalen, wie sie unter 8.c beschrieben sind.

#### Beispiel 8.b.4

Man verfährt, wie unter dem Verfahrensprinzip 8.b.1 beschrieben, unter Anwendung einer Eingangstemperatur des Gases (G) von 180°C und einer Fließbettemperatur (= Reaktionstemperatur) von 130°C. Man erhält ein Granulat mit der Zusammensetzung und den Qualitätsmerkmalen, wie sie unter 8.c beschrieben sind.

#### Beispiel 8.b.5

Man verfährt, wie unter dem Verfahrensprinzip 8.b.1 beschrieben, unter Anwendung einer Eingangstemperatur des Gases (G) von 200°C und einer Fließbettemperatur (= Reaktionstemperatur) von 155°C. Man erhält ein Granulat mit der Zusammensetzung und den Qualitätsmerkmalen, wie sie unter 8.c beschrieben sind.

### 8.c Ergebnisse

Anhand der Analysen von Granulatproben, die während des Einsprühens der schwefelsauren Lösung oder Suspension der Sulfonyl-Ausgangsverbindung aus dem Fließbett entnommen wurden, wurde festgestellt, daß die Veresterungsreaktion (Sulfatierung) während des Einsprühens spontan und praktisch vollständig abläuft,

so daß eine Nachreaktionszeit ("Temperzeit") nicht erforderlich ist. Dies wurde zusätzlich bestätigt durch Analysen von Granulatproben, die im Fließbett bei der gewählten Reaktionstemperatur einer "Temperzeit" von bis zu 4 Stunden unterworfen waren und deren Produktzusammensetzung sich hinsichtlich des Gehaltes an Sulfato-Endprodukt nicht mehr veränderte. Die Granulate der Beispiele 8.b.2 bis 8.b.5 enthielten durchschnittlich

92-96 % 2-Methoxy-5-methyl-4-(β-sulfatoethylsulfonyl)-anilin,
< 0,4 % 2-Methoxy-5-methyl-4-(β-hydroxyethylsulfonyl)-1-acetylaminobenzol und
2-5 % 2-Methoxy-5-methyl-4-(β-hydroxyethylsulfonyl)-anilin sowie
2-3 % Nebenprodukte, die in der technischen Ausgangsverbindung synthesebedingt enthalten waren.

Die praktisch staubfreien Granulate zeichnen sich gegenüber Produkten, die in Kontakttrocknern, beispielsweise einer Trockenpfanne, hergestellt wurden, durch eine höhere Lösegeschwindigkeit in wäßriger Suspension bei Zugabe von calcinierter Soda bis zu einem pH-Wert von 7 aus.

**9. Herstellung von 2-Methoxy-5-(β-sulfatoethylsulfonyl)-anilin**

**9-a Herstellung der Reaktionsgemische**

Man rührt 2-Methoxy-5-(β-hydroxyethylsulfonyl)-1-acetylamino-benzol technisch wasserfeucht oder technisch trocken in solch einer Menge wäßriger Schwefelsäure mit Prozentgehalten von 20 bis 95 % an, daß das Molverhältnis zwischen Sulfonylverbindung und Schwefelsäure beispielsweise 1:1,05 oder 1:1,1 beträgt. Man erhält bei beispielsweise 110-115°C eine Lösung oder bei 20 bis 25°C eine Suspension, die in den Konvektionstrockner eingespeist werden.

Bevorzugt wird mit Reaktionsgemischen garbeitet, die 50 bis 65 % 2-Methoxy-5-(β-hydroxyethylsulfonyl)-1-acetylamino -benzol und 19 bis 26 % Schwefelsäure (als 100 %ig gerechnet) enthalten.

**9.b Verfahrensbeispiele**

**9.b.1 Verfahrensprinzip**

In einem Wirbelschichtsprühgranulator (beispielsweise gemäß Figur 1) baut man mit dem Gas (G) und mit Feingut des Endproduktes, nämlich 2-Methoxy-5-(β-sulfatoethylsulfonyl)-anilin mit einer Korngröße von z.B. kleiner oder gleich 100 μm, ein Fließbett auf. Die Eingangstemperatur des Gases (G) wählt man beispielsweise wie in den Beispielen 9.b.2 bis 9.b.5 beschrieben und hält die Fließbettemperatur (= Reaktionstemperatur), wie in den Beispielen 9.b.2 bis 9.b.5 beschrieben, konstant, indem man stetig eine der unter 9.a hergestellten Lösungen oder suspensionen der Sulfonyl-Ausgangsverbindung in das Fließbett einsprüht. Im Fließbett entsteht hierbei ein Granulat, dessen Korngröße man durch Einschleusen von Feingut des Sulfato-Endproduktes und-/oder durch Zerkleinern mittels Zerhacker (25) steuert und so ein Granulat von beispielsweise 100 bis 800 μm oder 100 bis 2000 μm oder 100 bis 3000 μm erzeugt.

**Beispiel 9.b.2**

Man verfährt, wie unter dem Verfahrenspriznip 9.b.1 beschrieben, unter Anwendung einer Eingangstemperatur des Gases (G) von 200°C und einer Fließbettemperatur (= Reaktionstemperatur) von 150°C. Man erhält ein Granulat mit der Zusammensetzung und den Qualitätsmerkmalen, wie sie unter 9.c. beschrieben sind.

**Beispiel 9.b.3**

Man verfährt, wie unter dem Verfahrensprinzip 9.b.1 beschrieben, unter Anwendung einer Eingangstemperatur des Gases (G) von 220°C und einer Fließbettemperatur (= Reaktionstemperatur) von 165°C. Man erhält ein Granulat mit der Zusammensetzung und den Qualitätsmerkmalen, wie sie unter 9.c beschrieben sind.

**Beispiel 9.b.4**

Man verfährt, wie unter dem Verfahrensprinzip 9.b.1 beschrieben, unter Anwendung einer Eingangstemperatur des Gases (G) von 180°C und einer Fließbettemperatur (= Reaktionstemperatur) von 140°C. Man erhält ein Granulat mit der Zusammensetzung und den Qualitätsmerkmalen, wie sie unter 9.c beschrieben sind.

**Beispiel 9.b.5**

Man verfährt, wie unter dem Verfahrensprinzip 9.b.1 beschrieben, unter Anwendung einer Eingangstemperatur des Gases (G) von 200°C und einer Fließbettemperatur (= Reaktionstemperatur) von 130°C. Man erhält ein Granulat mit der Zusammensetzung und den Qualitätsmerkmalen, wie sie unter 9.c beschrieben sind.

**9.c Ergebnisse**

Anhand der Analysen von Granulatproben, die während des Einsprühens der schwefelsauren Lösung oder Suspension der Sulfonyl-Ausgangsverbindung aus dem Fließbett entnommen wurden, wurde festgestellt, daß die Veresterungsreaktion (Sulfatierung) während des Einsprühens spontan und praktisch vollständig abläuft, so daß eine Nachreaktionszeit ("Temperzeit") nicht erforderlich ist. Dies wurde zusätzlich bestätigt durch Analysen von Granulatproben, die im Fließbett bei der gewählten Reaktiontemperatur einer "Temperzeit" von bis zu 3 Stunden unterworfen waren und deren Produktzusammensetzung sich hinsichtlich des Gehaltes an Sulfato-Endprodukt nicht mehr veränderte.

Die Granulate der Beispiele 9.b.2 bis 9.b.5 enthielten durchschnittlich

94-96 % 2-Methoxy-5-(sulfatoethylsulfonyl)-anilin

1-2,5 % 2-Methoxy-5-(β-hydroxyethylsulfonyl)-anilin

3,5 % Nebenprodukte, die in der technischen Ausgangsverbindung synthesebedingt enthalten waren, in unveränderter Form.

Die praktisch staubfreien Granulate zeichnen sich gegenüber Produkten, die in Kontakttrocknern, beispielsweise einer Trockenpfanne, hergestellt wurden, durch eine höhere Lösegeschwindigkeit in wäßriger Suspension bei Zugabe von calcinierter Soda bis zu einem pH-Wert von 7 aus.

**Patentansprüche**

1. Verfahren zur Herstellung einer Aminoaryl-β-sulfatoethylsulfon-Verbindung durch Umsetzung einer Aminoaryl-β-hydroxyethylsulfon-Verbindung oder deren Acylamino-Derivat mit Schwefelsäure, dadurch gekennzeichnet, daß man eine Lösung oder Suspension oder Paste dieser Aminoaryl-β-hydroxyethylsulfon-Verbindung bzw. Deren Acylamino-Verbindung in 100 %iger Schwefelsäure oder in wäßriger Schwefelsäure mit einem Wassergehalt von bis zu 80 Gew.-% im Molverhältnis der β-Hydroxyethylsulfonyl-Ausgangsverbindung zu $H_2SO_4$ von 1:1 bis 1:1,05 nach deren Herstellung in den heißen Gasstrom eines Wirbelschichtsprühgranulators oder Sprühtrockners oder Sprühtrockners mit integriertem Fließbett einsprüht und darin die Trocknung, die Veresterungsreaktion und die Hydrolyse einer gegebenenfalls vorhandenen Acylamino-Gruppe synchron bei einer Temperatur zwischen 100 und 200°C durchführt.

2. Verfahren nach Anspruch 1, dadurch gekennzeichnet, daß man die Umsetzung bei einer Temperatur zwischen 110 und 180°C durchführt.

3. Verfahren nach Anspruch 1 oder 2, dadurch gekennzeichnet, daß die Schwefelsäure eine wäßrige Schwefelsäure ist.

4. Verfahren nach einem oder mehreren der Ansprüche 1 bis 3, dadurch gekennzeichnet, daß die Trocknung und Umsetzungen in einem Wirbelschichtsprühgranulator durchgeführt werden, bei dem die Wirbelschicht pneumatisch und/oder mechanisch erzeugt wird.

5. Verfahren nach einem oder mehreren der Ansprüche 1 bis 3, dadurch gekennzeichnet, daß die Trocknung und Umsetzungen in einem Sprühturm mit einer Zerstäuberscheibe oder mit Zerstäuberdüse(n) durchgeführt werden.

6. Verfahren nach einem oder mehreren der Ansprüche 1 bis 3, dadurch gekennzeichnet, daß die Trocknung und Umsetzungen in einem Sprühtrockner mit integriertem Fließbett durchgeführt werden.

7. Verfahren nach einem oder mehreren der Ansprüche 1 bis 6, dadurch gekennzeichnet, daß man eine β-Hydroxyethylsulfonyl-Verbindung entsprechend der allgemeinen Formel (2)

$$(HO-CH_2-CH_2-SO_2)_m—A—\underset{R}{\underset{|}{N}}—H \qquad (2)$$

in welcher m die Zahl 1 oder 2 bedeutet, A ein Phenylen-oder Naphthylenrest ist, die durch 1, 2 oder 3 Substituenten aus der Gruppe Alkyl von 1 bis 4 C-Atomen, Alkoxy von 1 bis 4 C-Atomen, Halogen, Carboxy und Hydroxy substituiert sein können, und R für ein Wasserstoffatom oder eine Acylgruppe steht, zu einer Schwefelsäurehalbesterverbindung entsprechend der allgemeinen Formel (1)

$$(HO_3SO - CH_2 - CH_2 - SO_2)_m - A - NH_2 \quad (1)$$

in welcher A und m die obengenannten Bedeutungen haben, umsetzt.

8. Verfahren nach einem oder mehreren der Ansprüche 1 bis 6, dadurch gekennzeichnet, daß man 4-(β-Hydroxyethylsulfonyl)-1-acetylamino-benzol zu 4-(β-Sulfatoethylsulfonyl)-anilin umsetzt.

9. Verfahren nach einem oder mehreren der Ansprüche 1 bis 6, dadurch gekennzeichnet, daß man 3-(β-Hydroxyethylsulfonyl)-anilin zu 3-(β-Sulfatoethyl-sulfonyl)-anilin umsetzt.

10. Verfahren nach einem oder mehreren der Ansprüche 1 bis 6, dadurch gekennzeichnet, daß man 2-Brom-4-(β-hydroxyethylsulfonyl)-anilin zu 2-Brom-4-(β-sulfatoethylsulfonyl)-anilin umsetzt.

11. Verfahren nach einem oder mehreren der Ansprüche 1 bis 6, dadurch gekennzeichnet, daß man 2-Methoxy-5-methyl-4-(β-hydroxyethylsulfonyl)-1-acetylamino-benzol zu 2-Methoxy-5-methyl-4-(β-sulfatoethylsulfonyl)-anilin umsetzt.

12. Verfahren nach einem oder mehreren der Ansprüche 1 bis 6, dadurch gekennzeichnet, daß man 2,5-Dimethoxy-4-(β-hydroxyethylsulfonyl)-1-acetylamino-benzol zu 2,5-Dimethoxy-4-(β-sulfatoethylsulfonyl)-anilin umsetzt.

13. Verfähren nach einem oder mehreren der Ansprüche 1 bis 6, dadurch gekennzeichnet, daß man 2-Methoxy-5-(β-hydroxyethylsulfonyl)-1-acetylamino-benzol zu 2-Methoxy-5-(β-sulfatoethylsulfonyl)-anilin umsetzt.

## Claims

1. A process for the preparation of an aminoaryl β-sulfatoethyl sulfone compound by reaction of an aminoaryl β-hydroxyethyl sulfone compound or an acylamino derivative thereof with sulfuric acid, which comprises spraying a solution or suspension or paste of this aminoaryl β-hydroxyethyl sulfone compound or the acylamino compound thereof in 100% sulfuric acid or in aqueous sulfuric acid having a water content of up to 80% by weight in a molar ratio of the β-hydroxyethylsulfonyl starting compound to $H_2SO_4$ of 1:1 to 1:1.05 after its preparation into the hot gas stream of a fluidized bed spray granulator or a spray dryer or a spray dryer having an integrated fluid bed and carrying out the drying, the esterification reaction and the hydrolysis of any acylamino group which may be present synchronously at a temperature between 100 and 200°C.

2. The process as claimed in claim 1, wherein the reaction is carried out at a temperature between 110 and 180°C.

3. The process as claimed in claim 1 or 2, wherein the sulfuric acid is an aqueous sulfuric acid.

4. The process as claimed in one or more of claims 1 to 3, wherein the drying and the reactions are carried out in a fluidized bed spray granulator in which the fluidized bed is produced pneumatically and/or mechanically.

5. The process as claimed in one or more of claims 1 to 3, wherein the drying and the reactions are carried out in a spray tower containing an atomizing disk or an atomizing nozzle or nozzles.

6. The process as claimed in one or more of claims 1 to 3, wherein the drying and the reactions are carried out in a spray dryer having an integrated fluid bed.

7. The process as claimed in one or more of claims 1 to 6, wherein a β-hydroxyethylsulfonyl compound conforming to the general formula (2)

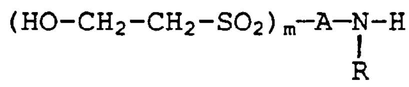

(2)

in which m denotes the number 1 or 2, A is a phenylene or naphthylene radical, both of which can be substituted by 1, 2 or 3 substituents from the group consisting of alkyl of 1 to 4 carbon atoms, alkoxy of 1 to 4 carbon atoms, halogen, carboxyl and hydroxyl, and R stands for a hydrogen atom or an acyl group, is converted to a sulfuric acid half ester compound conforming to the general formula (1)

$$(HO_3SO - CH_2 - CH_2 - SO_2)_m - A - NH_2 \quad (1)$$

in which A and m have the abovementioned meanings.

8. The process as claimed in one or more of claims 1 to 6, wherein 4-(β-hydroxyethylsulfonyl)-acetanilide is converted to 4-(β-sulfatoethylsulfonyl)-aniline.

9. The process as claimed in one or more of claims 1 to 6, wherein 3-(β-hydroxyethylsulfonyl)aniline is converted to 3-(β-sulfatoethylsulfonyl)-aniline.

10. The process as claimed in one or more of claims 1 to 6, wherein 2-bromo-4-(β-hydroxyethylsulfonyl)-aniline is converted to 2-bromo-4-(β-sulfatoethylsulfonyl)-aniline.

11. The process as claimed in one or more of claims 1 to 6, wherein 2-methoxy-5-methyl-4-(β-hydroxyethylsulfonyl)-acetanilide is converted to 2-methoxy-5-methyl-4-(β-sulfatoethylsulfonyl)-aniline.

12. The process as claimed in one or more of claims 1 to 6, wherein 2,5-dimethoxy-4-(β-hydroxyethylsulfonyl)-acetanilide is converted to 2,5-dimethoxy-4-(β-sulfatoethylsulfonyl)-aniline.

13. The process as claimed in one or more of claims 1 to 6, wherein 2-methoxy-5-(β-hydroxyethylsulfonyl)-acetanilide is converted to 2-methoxy-5-(β-sulfatoethylsulfonyl)-aniline.

## Revendications

1. Procédé pour préparer une (β-sulfatoéthyl-sulfonyl)-arylamine par réaction d'une (β-hydroxyéthyl-sulfonyl)-arylamine, ou du composé acylaminé correspondant, avec l'acide sulfurique, procédé caractérisé en ce qu'on injecte par pulvérisation une solution, une suspension ou une pâte de cette (β-hydroxyéthyl-sulfonyl)-arylamine, ou du composé acylaminé correspondant, dans de l'acide sulfurique à 100 % ou dans un acide sulfurique aqueux dont la teneur en eau peut aller jusqu'à 80 % en poids, avec un rapport molaire du composé β-hydroxyéthyl-sulfonylique de départ à $H_2SO_4$ compris entre 1:1 et 1:1,05, après leur préparation, dans le courant gazeux chaud d'un granulateur pulvérisateur à couche tourbillonnaire ou d'un séchoir par pulvérisation ou d'un séchoir par pulvérisation à lit fluidisé intégré, et on effectue dans cet appareil, en même temps, le séchage, la réaction d'estérification et l'hydrolyse d'un éventuel radical acylamino, à une température comprise entre 100 et 200 °C.

2. Procédé selon la revendication 1 caractérisé en ce qu'on effectue la réaction à une température comprise entre 110 et 180 °C.

3. Procédé selon l'une des revendications 1 et 2 caractérisé en ce que l'acide sulfurique est un acide sulfurique aqueux.

4. Procédé selon une ou plusieurs des revendications 1 à 3, caractérisé en ce que le séchage et les réactions sont effectués dans un granulateur pulvérisateur à couche tourbillonnaire dans lequel la couche tourbillonnaire est créée pneumatiquement et/ou mécaniquement.

5. Procédé selon une ou plusieurs des revendications 1 à 3, caractérisé en ce que le séchage et les réactions sont effectués dans une tour de pulvérisation avec un disque pulvérisateur ou avec une ou des buses pulvérisatrices.

**6.** Procédé selon une ou plusieurs des revendications 1 à 3, caractérisé en ce que le séchage et les réactions sont effectués dans un séchoir par pulvérisation à lit fluidisé intégré.

**7.** Procédé selon une ou plusieurs des revendications 1 à 6, caractérisé en ce qu'on fait réagir un composé β-hydroxyéthyl-sulfonylique répondant à la formule générale 2 :

$$(HO-CH_2-CH_2-SO_2)_m-A-\underset{\displaystyle R}{\underset{|}{N}}-H \qquad (2)$$

dans laquelle m désigne le nombre 1 ou le nombre 2, A représente un radical phénylène ou naphtylène éventuellement porteur d'un, de deux ou de trois substituants pris dans l'ensemble constitué par les alkyles contenant de 1 à 4 atomes de carbone, les alcoxy contenant de 1 à 4 atomes de carbone, les halogènes, le carboxy et l'hydroxy, et R représente un atome d'hydrogène ou un radical acyle. de manière à obtenir un hémi-ester sulfurique correspondant qui répond à la formule générale 1 :

$$(HO_3SO - CH_2 - CH_2 - SO_2)_m - A - NH_2 \qquad (1)$$

dans laquelle A et m ont les significations qui leur ont été données ci-dessus.

**8.** Procédé selon une ou plusieurs des revendications 1 à 6, caractérisé en ce qu'on fait réagir le 4-(β-hydroxyéthyl-sulfonyl)-1-acétylamino-benzène pour obtenir la 4-(β-sulfatoéthyl-sulfonyl)-aniline.

**9.** Procédé selon une ou plusieurs des revendications 1 à 6, caractérisé en ce qu'on fait réagir la 3-(β-hydroxyéthyl-sulfonyl)-aniline pour obtenir la 3-(β-sulfatoéthyl-sulfonyl)-aniline.

**10.** Procédé selon une ou plusieurs des revendications 1 à 6, caractérisé en ce qu'on fait réagir la 2-bromo-4-(β-hydroxyéthyl-sulfonyl)-aniline pour obtenir la 2-bromo-4-(β-sulfatoéthyl-sulfonyl)-aniline.

**11.** Procédé selon une ou plusieurs des revendications 1 à 6, caractérisé en ce qu'on fait réagir le 2-méthoxy-5-méthyl-4-(β-hydroxyéthyl-sulfonyl)-1-acétylamino-benzène pour obtenir la 2-méthoxy-5-méthyl-4-(β-sulfatoéthylsulfonyl)-aniline.

**12.** Procédé selon une ou plusieurs des revendications 1 à 6, caractérisé en ce qu'on fait réagir le 2,5-diméthoxy-4-(β-hydroxyéthyl-sulfonyl)-1-acétylamino-benzène pour obtenir la 2,5-diméthoxy-4-(β-sulfatoéthyl-sulfonyl)-aniline.

**13.** Procédé selon une ou plusieurs des revendications 1 à 6, caractérisé en ce qu'on fait réagir le 2-méthoxy-5-(β-hydroxyéthyl-sulfonyl)-1-acétylamino-benzène pour obtenir la 2-méthoxy-5-(β-sulfatoéthyl-sulfonyl)-aniline.

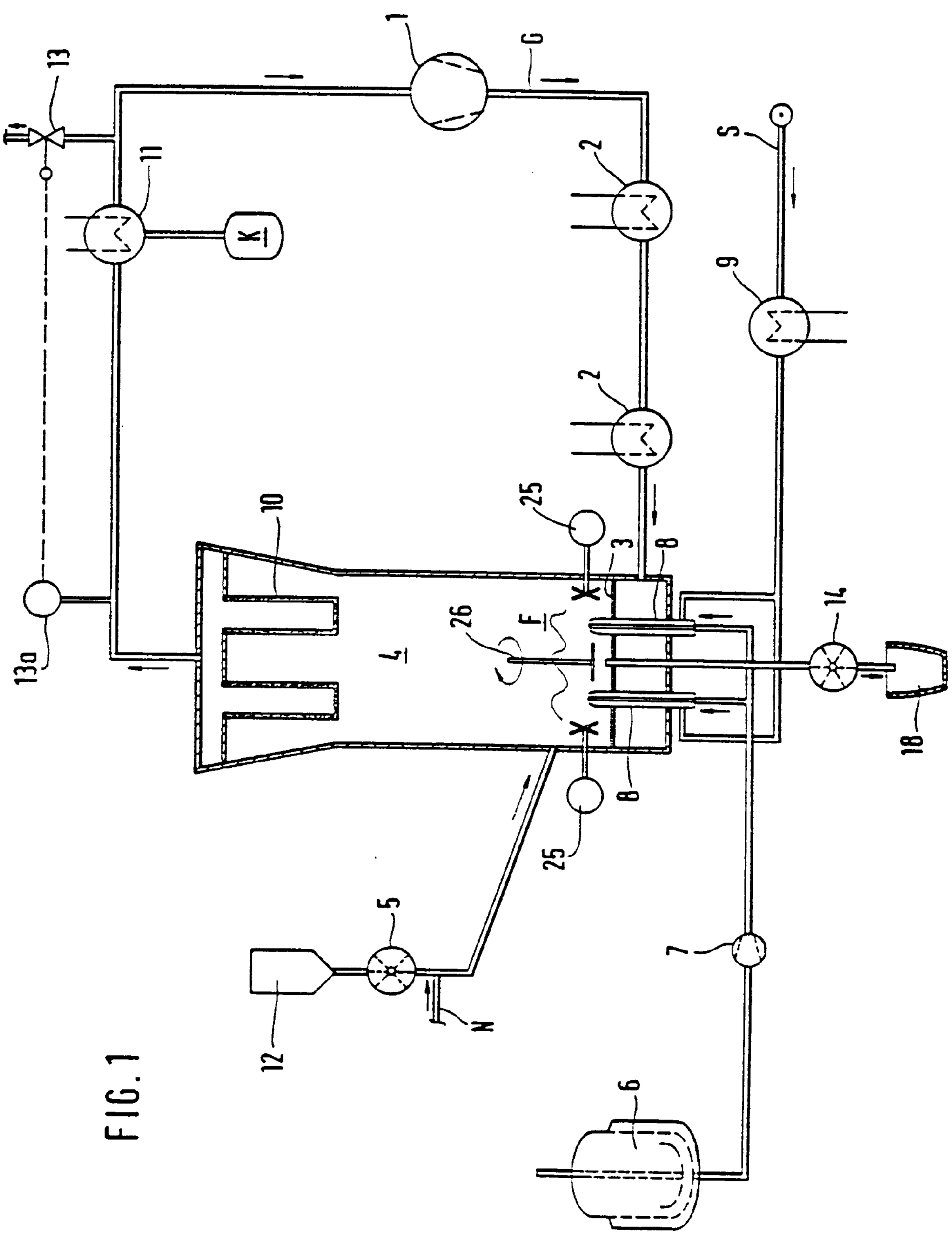


FIG. 1

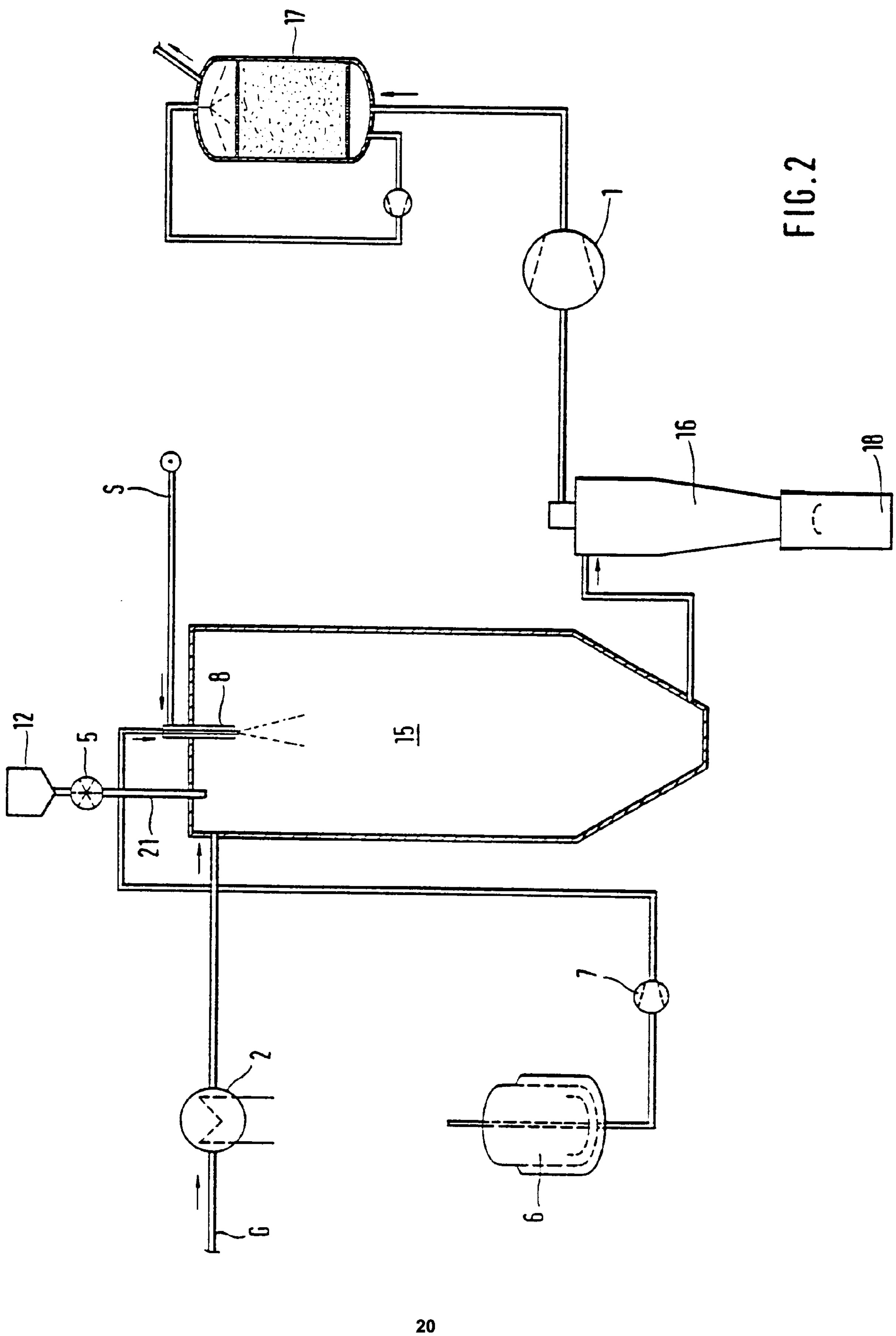


FIG.2

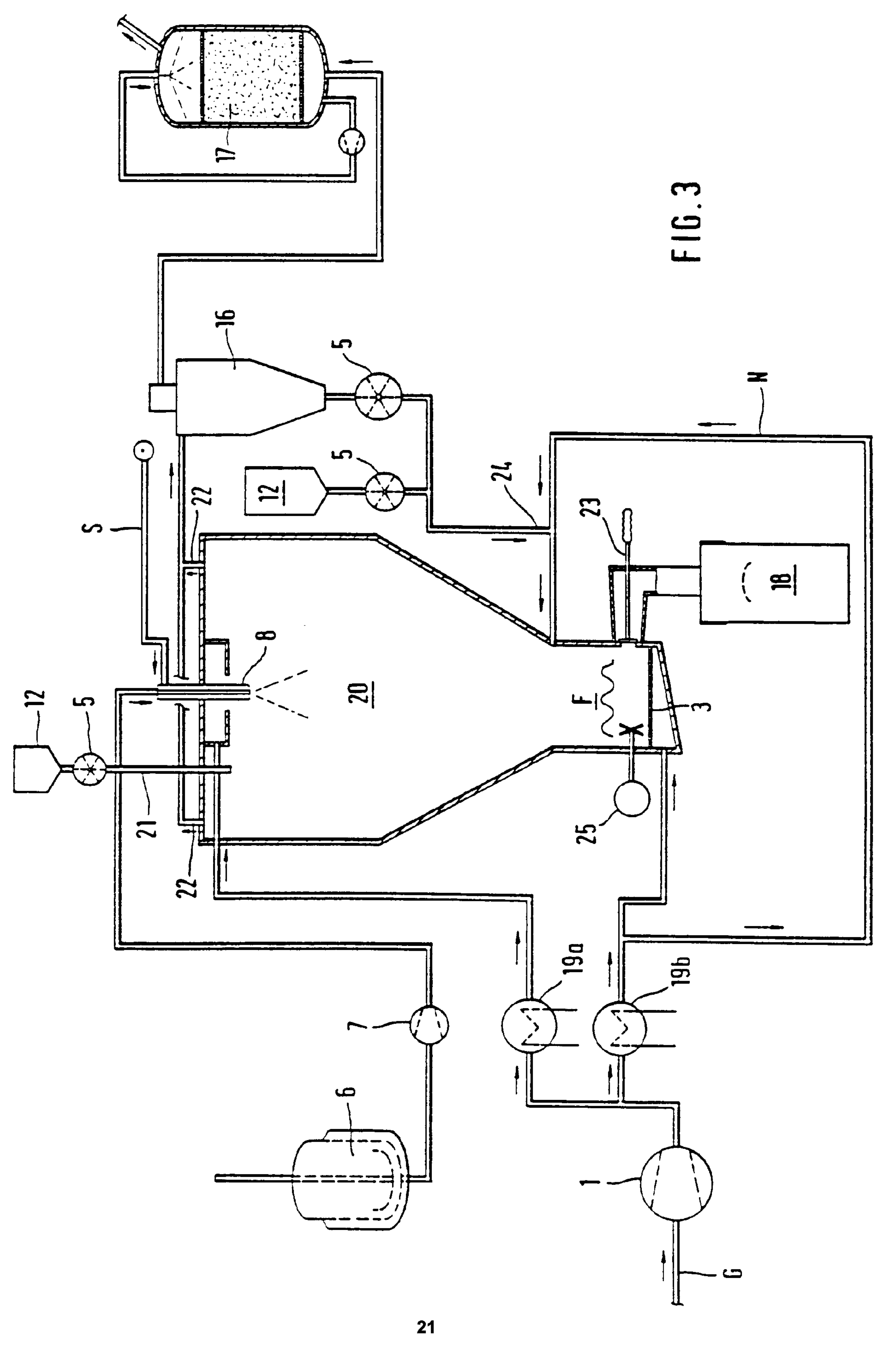


FIG. 3